# EUROPEAN PATENT SPECIFICATION

(11) **EP 4 240 471 B1**
(45) Date of publication and mention of the grant of the patent: **20.05.2026**
(21) Application number: 21890287.2
(22) Date of filing: 09.11.2021
(51) Int. Cl.: A61N 1/05, A61N 1/362, A61N 1/372, A61N 1/378, A61N 1/375

(54) **SYSTEMS FOR WIRELESS ENDOCARDIAL STIMULATION OF THE LEFT VENTRICULAR SEPTAL WALL**
SYSTEME ZUR DRAHTLOSEN ENDOKARDIALEN STIMULATION DER LINKSVENTRIKULÄREN SEPTUMWAND
SYSTÈMES DE STIMULATION ENDOCARDIAQUE SANS FIL DE LA PAROI SEPTALE VENTRICULAIRE GAUCHE

(30) Priority: 09.11.2020 US 202063111512 P
(43) Date of publication of application: 13.09.2023
(73) Proprietor: EBR Systems, Inc., Sunnyvale, CA 94085 (US)
(72) Inventor: WILLIS, Parker, Sunnyvale, California 94085 (US); FAYRAM, Timothy A., Sunnyvale, California 94085 (US); WILL, Allan, Sunnyvale, California 94085 (US); RILEY, Richard, Sunnyvale, California 94085 (US); SAM, John P., Sunnyvale, California 94085 (US)
(74) Representative: Mathys & Squire
(86) International application number: PCT/US2021/058645
(87) International publication number: WO 2022/099201

(56) References cited:
- WO-A1-2009/006531
- WO-A1-2009/006531
- JP-B2- 5 111 116
- US-A1- 2005 288 727
- US-A1- 2005 288 727
- US-A1- 2007 060 961
- US-A1- 2009 018 599
- US-A1- 2009 326 601
- US-A1- 2010 063 562
- US-A1- 2011 276 102
- US-A1- 2011 276 102
- US-A1- 2015 151 116
- US-A1- 2015 173 794
- US-A1- 2020 338 356
- US-A1- 2020 338 356
- US-B2- 8 644 934

## Description

### TECHNICAL FIELD

The present technology generally relates to systems for stimulating cardiac tissue and, more particularly, to systems for wirelessly stimulating (e.g., pacing) the left ventricular septal wall of a human patient.

### BACKGROUND

There are two branches of the bundle of His: the left bundle branch and the right bundle branch, both of which are located along the interventricular septum. The left bundle branch further divides into the left anterior fascicles and the left posterior fascicles. These structures lead to a network of thin filaments known as Purkinje fibers, and play an integral role in the electrical conduction system of the heart by transmitting cardiac action potentials to the Purkinje fibers.

When a bundle branch or fascicle becomes injured (e.g., by underlying heart disease, myocardial infarction, or cardiac surgery), it may cease to conduct electrical impulses appropriately, resulting in altered pathways for ventricular depolarization. This condition is known as a bundle branch block.

Pacing on the left ventricular septa! wall has been viewed theoretically as a way of directly stimulating the conduction system and creating a normalizing effect on ventricular depolarization. This effect could restore normal ventricular synchrony and increase a cardiac pump function from the diseased state.

In US 8,644,934 an implantable cardiac tissue excitation system which includes an implantable pacing controller unit with a pulse generation circuit is described. The system also includes a lead with a lead body extending between a proximal lead end attachable to the pacing controller unit and a distal lead end configured to be implanted within a heart. A lead conductor extends within the lead body. The system also includes a transmitter assembly located near the distal lead end that is electrically connected to the pulse generation circuit through the lead conductor to wirelessly transmit pacing control information and pacing energy. The system also includes a leadless electrode assembly configured to be implanted within the heart that includes a receiver to receive the wireless transmission, a charge storage unit to store the charge energy, and an electrical stimulation circuit to deliver an electrical stimulus to cardiac tissue using the pacing control information and the charge energy.

In JP-B2-5111116, systems including an implantable receiver-stimulator and an implantable controller-transmitter are described, for leadless electrical stimulation of body tissues. Cardiac pacing and arrhythmia control is accomplished with one or more implantable receiver-stimulators and an external or implantable controller-transmitter. Systems are implanted by testing external or implantable devices at different tissue sites, observing physiologic and device responses, and selecting sites with preferred performance for implanting the systems. In these systems, a controller-transmitter is activated at a remote tissue location to transmit/deliver acoustic energy through the body to a receiver-stimulator at a target tissue location. The receiver-stimulator converts the acoustic energy to electrical energy for electrical stimulation of the body tissue. The tissue locations(s) can be optimized by moving either or both of the controller-transmitter and the receiver-stimulator to determine the best patient and device responses.

In US 2011/0276102 a pacing system is described which includes a controller operable to provide control signals indicating desired pacing signals, a pulse generator connected to the controller and operable to receive the control signals and to generate the desired pacing signals based on the control signals, at least one lead electrically connected to the pulse generator and extending into a user's heart and operable to provide the pacing signals to the heart, at least one electrode positioned in the user's heart and electrically connected to the at least one lead, the at least one electrode in contact with the user's heart and operable to stimulate the heart based on the pacing signals; and a transceiver, in communication with the pulse generator and operable to selectively transmit the pacing signals to the electrode wirelessly. The transceiver is controlled by the controller to transmit the pacing signals when pacing signals are not received by the electrode from the at least one lead. The lead may include multiple leads held together in a sugar moiety as a unitary body for insertion into the heart. Once in the heart, the sugar moiety dissolves to allow the leads to separate for implantation at different points in the heart.

In US 2009/0326601 receiver-stimulators which comprise a nearly isotropic transducer assembly, demodulator circuitry, and at least two tissue contacting electrodes are discussed. Use of near isotropic transducers allows the devices to be implanted with less concern regarding the orientation relative to an acoustic energy source. Transducers or transducer elements having relatively small sizes, typically less than ½ the wavelength of the acoustic source, enhance isotropy. The use of single crystal piezoelectric materials enhances sensitivity.

WO 2009/006531 describes methods and systems for minimizing energy utilization for tissue stimulation using implantable microstimulators are disclosed. Microstimulators are designed to minimize the formation of fibrotic tissue, which results in increased energy consumption during tissue stimulation. Furthermore, by using microstimulators in combination with electrodes with small surface areas that are 1mm² or less, tissue can be stimulated using lower energies. Small electrode surface areas achieve high current densities and this combined with the small fibrotic cap thickness improves energy utilization in implanted tissue microstimulators. In an example, the micro stimulator converts acoustic energy that is received from an acoustic transmitter into electrical energy and the electrical signals are used to stimulate the tissue using optimized energy utilization.

In US 2007/0060961, methods and apparatus for determining an endocardial implantation site for implanting an electrode, such as a leadless stimulation electrode are described. An example includes delivering sufficient electrical energy for initiation of cardiac activation to a plurality of different test locations at the heart of a patient, and determining hemodynamic responses in reaction to that the stimulus delivered to the different test locations. This method further includes identifying an implantation site for implanting the electrode by selecting at least one of the test locations corresponding to a favorable hemodynamic response.

In US 2005/0288727 a system for monitoring heart performance is described, comprising a plurality of sensing devices configured to attach to a patient's heart tissue and a controller. Each sensing device comprises a sensor configured to detect physiological data relating to heart contractility and a wireless transmitter configured to transmit data detected by the sensor. The controller comprises a receiver configured to receive the detected data transmitted by the plurality of sensing devices and a processor configured to analyze the received data.

US 2020/0338356 describes a leadless cardiac pacemaker device is configured to provide HIS bundle pacing and contains a housing having a tip, a first electrode arranged on the housing in the vicinity of the tip, the first electrode being configured to engage with intracardiac tissue, and a second electrode arranged on the housing at a distance from the tip of the housing. A processor is enclosed in the housing and operatively connected to the first electrode and the second electrode. The processor is configured to process a reception signal received by at least one of the first electrode and the second electrode and to generate a pacing signal to be emitted using at least one of the first electrode and the second electrode.

US 2015/0151116 discloses a method of implanting a lead in the left heart cavity including introducing the lead into the right heart cavity. The lead includes a lead body having a deformable sheath, a proximal end having an electrical connector, a distal end including a projecting helical screw electrode, and a conductor extending along the sheath, electrically connecting the electrical connector and the helical screw. The method further includes positioning the distal end of the lead to abut a septum wall between the right and left heart cavity. The electrical connector is connected to an RF puncture generator and RF energy is applied to the screw while providing rotational movement to the screw for advancement through the septum wall. The method further includes positioning the screw at a target stimulation site in the left heart cavity and providing rotational movement to the screw to anchor the lead at the target site.

US 2015/0173794 discloses devices and methods for performing a transseptal puncture procedure using a device which includes either an untampered or tapered blunt end cannula disposed in an introducer carrying a sharp guidewire disposed longitudinally through the lumen of the blunt cannula, and a blunt end dilator wherein the guidewire is flexible and has an atraumatic shape at its tip. The cannula gives the more flexible introducer a defined shape and steerabilty allowing an ordinarily skilled physician to easily access a selected location on the septa! wall of the heart for transseptal puncture and introducer placement thereacross without employing an exposed sharp end needle during the procedure.

### BRIEF DESCRIPTION OF THE DRAWINGS

Many aspects of the present disclosure can be better understood with reference to the following drawings. The components in the drawings are not necessarily to scale. Instead, emphasis is placed on clearly illustrating the principles of the present disclosure.
Figure 1 is a schematic diagram of a tissue stimulation system in accordance with embodiments of the present technology.
Figure 2 is a side view of a pair of receiver-stimulators secured to a septal wall of a heart of a patient and within a left ventricle of the heart in accordance with embodiments of the present technology.
Figures 3A and 3B are a side view and a transverse cross-sectional view, respectively, of a receiver-stimulator secured to the septal wall within the left ventricle in accordance with embodiments of the present technology.
Figure 4 is a side view of a receiver-stimulator positioned in the left ventricle in accordance with embodiments of the present technology.
Figure 5A is an isometric view of a receiver-stimulator, and Figure 5B is a side view of the receiver-stimulator secured to the septal wall within the left ventricle, in accordance with embodiments of the present technology.
Figure 6A is a side view of a receiver stimulator, and Figure 6B is a front view from inside the right ventricle of the receiver-stimulator secured to the septal wall, in accordance with embodiments of the present technology.
Figure 7A is a side view of a receiver stimulator, and Figure 7B is a front view from inside the right ventricle of the receiver-stimulator secured to the septal wall, in accordance with embodiments of the present technology.
Figure 8A is a side view of a receiver stimulator, and Figure 8B is a front view from inside the right ventricle of the receiver-stimulator secured to the septal wall, in accordance with embodiments of the present technology.
Figure 9 is a side view of a receiver-stimulator secured to the septal wall in accordance with embodiments of the present technology.
Figure 10 is a side view of a receiver-stimulator secured to the septal wall in accordance with embodiments of the present technology.
Figure 11A is a side view of a distal portion of a delivery system configured to implant a receiver-stimulator within the heart of a patient in accordance with embodiments of the present technology. Figure 11B is an enlarged side view of the distal portion of the delivery system 11A in accordance with embodiments of the present technology.
Figure 12 is a side view of a distal portion of a delivery system configured to implant a receiver-stimulator within the heart of a patient in accordance with embodiments of the present technology.
Figure 13 is a side view of a portion of a delivery system configured to implant a receiver-stimulator within the heart of a patient in accordance with embodiments of the present technology.
Figure 14 is a side view of a portion of a delivery system configured to implant the receiver-stimulator of Figure 13 within the heart of a patient in accordance with embodiments of the present technology.
Figures 15A-15I and 15K are side views of a distal portion of a delivery system during different stages of a procedure to implant a receiver-stimulator within the septal wall of a heart of a patient in accordance with embodiments of the present technology. Figure 15J is a rear view from inside the left ventricle of the receiver-stimulator implanted at the septal wall in accordance with embodiments of the present technology.

### DETAILED DESCRIPTION

Aspects of the present disclosure are directed to systems for implanting stimulation assemblies (which can be referred to as receiver-stimulators, stimulation electrodes, pacing electrodes, and the like) at, in, and/or proximate to the septal wall (e.g., the left ventricular (LV) septal wall) of the heart of a patient, such as a human patient. In several of the embodiments described below, for example, a stimulation assembly includes a body, circuitry positioned at least partially within the body, an electrode, and an anchor coupled to the body. The anchor can be secured to the septal wall such that the body is positioned within the left ventricle of the heart and the electrode engages tissue of the septal wall. The circuitry can be configured to (i) receive acoustic energy from a remote wireless controller-transmitter and (ii) convert the acoustic energy to electrical energy. The electrode can deliver the electrical energy to the tissue of the septal wall to stimulate the tissue.

In some embodiments, the anchor can be secured to the septal wall via rotation of the anchor. In other embodiments, the anchor can be secured to the septal wall via a push-to-anchor method or via a pull-back-to-deploy and push-to-anchor method. The electrode can comprise one or more electrodes and, in some embodiments, can comprise an electrode array that is bipolar, tripolar, or quadripolar to accommodate the spatial nature of a particular septal wall pacing application. In some embodiments, the stimulation assembly includes programmable parameters for the array of electrodes including, for example, vectors, locations, and/or timing sequences configured to effectively stimulate the left bundle branch, the bundle of His, and/or other regions of the cardiac conduction system.

In some embodiments, a delivery system in accordance with the present technology for delivering a stimulation assembly can be configured to accommodate the tight radius turn required to access the conduction structures of the LV septal wall via an intravascular approach-for example, an intravascular approach comprising a puncture in the septum between the right atrium and the left atrium, through the left atrium, and across the mitral valve. For example, the delivery system can include a delivery sheath or catheter that includes a gland or other rotatable component that enables rotation of a distal end of the delivery sheath relative to the septal wall to facilitate placement of a stimulation assembly at the septal wall. Similarly, the delivery system can facilitate delivery of the stimulation assembly from an arterial approach through the aortic valve.

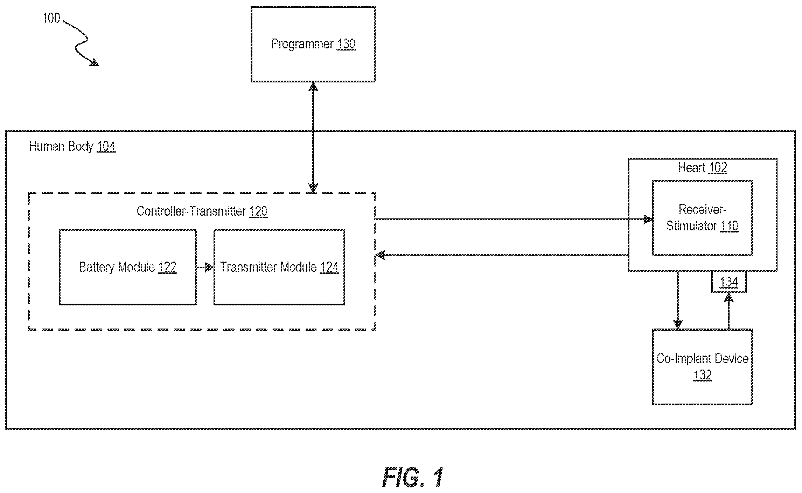

Specific details of several embodiments of the present technology are described herein with reference to Figures 1-15K. The present technology, however, can be practiced without some of these specific details. In some instances, well-known structures and techniques often associated with leadless tissue stimulation systems, cardiac pacing, electronic circuitry, acoustic and radiofrequency transmission and receipt, delivery systems and catheters, and the like, have not been shown in detail so as not to obscure the present technology. Moreover, although many of the embodiments are described below with respect to systems for left ventricular (LV) septal wall cardiac pacing, other applications and other embodiments in addition to those described herein are within the scope of the technology. For example, one of ordinary skill in the art will understand that one or more aspects of the present technology are applicable to other implantable devices configured to treat other areas of the human body.

The terminology used in the description presented below is intended to be interpreted in its broadest reasonable manner, even though it is being used in conjunction with a detailed description of certain specific embodiments of the disclosure. Certain terms can even be emphasized below; however, any terminology intended to be interpreted in any restricted manner will be overtly and specifically defined as such in this Detailed Description section.

The accompanying Figures depict embodiments of the present technology and are not intended to be limiting of its scope. The sizes of various depicted elements are not necessarily drawn to scale, and these various elements can be arbitrarily enlarged to improve legibility. Component details can be abstracted in the Figures to exclude details such as position of components and certain precise connections between such components when such details are unnecessary for a complete understanding of how to make and use the present technology. Many of the details, dimensions, angles, and other features shown in the Figures are merely illustrative of particular embodiments of the disclosure. Accordingly, other embodiments can have other details, dimensions, angles, and features without departing from the scope of the present technology.

With regard to the terms "distal" and "proximal" within this description, unless otherwise specified, the terms can reference a relative position of the portions of a catheter subsystem with reference to an operator and/or a location in the vasculature. Also, as used herein, the designations "rearward," "forward," "upward," "downward," and the like are not meant to limit the referenced component to a specific orientation. It will be appreciated that such designations refer to the orientation of the referenced component as illustrated in the drawings; the systems of the present technology can be used in any orientation suitable to the user.

The headings provided herein are for convenience only and should not be construed as limiting the subject matter disclosed.

### I. Selected Embodiments of Tissue Stimulation Systems

Figure 1 is a schematic diagram of a tissue stimulation system 100 ("system 100") in accordance with embodiments of the present technology. In the illustrated embodiment, the system 100 is configured to stimulate a heart 102 within a body 104 of a human patient. The system 100 can include one or more receiver-stimulators 110 (one shown in Figure 1; which can also be referred to as stimulators, stimulation assemblies, ultrasound receivers, stimulating electrodes, stimulation electrodes, pacing electrodes, acoustic receivers, and the like) in operable communication (e.g., wireless and/or radio communication) with a controller-transmitter 120 (which can also be referred to as an ultrasound transmitter, a pulse generator, an acoustic transmitter, and the like). The controller-transmitter 120 can include a battery module 122 and a transmitter module 124 operably coupled to and powered via the battery module 122. In some embodiments, both the receiver-stimulator 110 and the controller-transmitter 120 are configured to be implanted within the body 104 of the human patient. For example, the receiver-stimulator 110 can be implanted at and/or proximate the heart 102 (e.g., in the left ventricle, the right ventricle, or proximate area) for delivering stimulation pulses to the heart 102, while the controller-transmitter 120 can be positioned at another location remote from the heart 102 (e.g., in the chest area). In a particular embodiment, the receiver-stimulator 110 is positioned within the left ventricle and configured to stimulate endocardial tissue of the septal wall. The transmitter module 124 of the controller-transmitter 120 can direct energy (e.g., acoustic energy, ultrasound energy) toward the receiver-stimulator 110, which can receive the energy and deliver one or more electrical pulses (e.g., stimulation pulses, pacing pulses) to the heart 102.

In some embodiments, the system 100 can further include a programmer 130 in operable communication with the controller-transmitter 120. The programmer 130 can be positioned outside the body 104 and can be operable to program various parameters of the controller-transmitter 120 and/or to receive diagnostic information from the controller-transmitter 120. In some embodiments, the system 100 further includes a co-implant device 132 (e.g., an implantable cardioverter defibrillator (ICD) or pacemaker) coupled to pacing leads 134 for delivering stimulation pulses to one or more portions of the heart 102 other than the area stimulated by the receiver-stimulator 110. In other embodiments, the co-implant device 132 can be a leadless pacemaker which is implanted directly into the heart 102 to eliminate the need for separate pacing leads 134. The co-implant device 132 and the controller-transmitter 120 can operate in tandem and deliver stimulation signals to the heart 102 to cause a synchronized heartbeat. In some embodiments, the controller-transmitter 120 receives signals (e.g., electrocardiogram signals) from the heart 102 to determine information related to the heart 102, such as a heart rate, heart rhythm, including the output of the pacing leads 134 located in the heart 102. In some embodiments, the controller-transmitter 120 alternatively or additionally receives information (e.g., diagnostic signals) from the receiver-stimulator 110. The received signals can be used to adjust the ultrasound energy signals delivered to the receiver-stimulator 110.

The receiver-stimulator 110, the controller-transmitter 120, and/or the programmer 130 can include a machine-readable (e.g., computer-readable) or controller-readable medium containing instructions for generating, transmitting, and/or receiving suitable signals (e.g., stimulation signals, diagnostic signals). The receiver-stimulator 110, the controller-transmitter 120, and/or the programmer 130 can include one or more processor(s), memory unit(s), and/or input/output device(s). Accordingly, the process of providing stimulation signals and/or executing other associated functions can be performed by computer-executable instructions contained by, on, or in computer-readable media located at the receiver-stimulator 110, the controller-transmitter 120, and/or the programmer 130. Further, the receiver-stimulator 110, the controller-transmitter 120, and/or the programmer 130 can include dedicated hardware, firmware, and/or software for executing computer-executable instructions that, when executed, perform any one or more methods, processes, and/or sub-processes described herein. The dedicated hardware, firmware, and/or software also serve as "means for" performing the methods, processes, and/or sub-processes described herein.

**In** some embodiments, the system 100 can include several features generally similar or identical to those of the leadless tissue stimulation systems disclosed in (i) U.S. Patent 7,610,092, filed December 21, 2005, and titled "LEADLESS TISSUE STIMULATION SYSTEMS AND METHODS," (ii) U.S. Patent 8,315,701, filed September 4, 2009, and titled "LEADLESS TISSUE STIMULATION SYSTEMS AND METHODS," and/or (iii) U.S. Patent 8,718,773, filed May 23, 2007, and titled "OPTIMIZING ENERGY TRANSMISSION IN A LEADLESS TISSUE STIMULATION SYSTEM."

### II. Selected Embodiments of Receiver-Stimulators

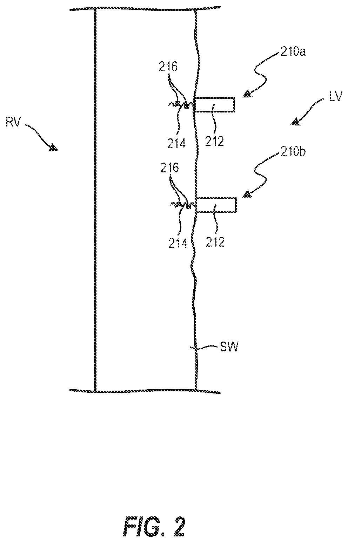

Figures 2-10 illustrate various receiver-stimulators configured in accordance with embodiments of the present technology. The receiver-stimulators can operate in the environment of Figure 1 and, in some embodiments, can be implantable within the left ventricle and/or configured to stimulate the septal wall of a human heart. For example, the receiver-stimulators can be implanted at the heart 102 and configured to receive acoustic energy (e.g., ultrasound energy) from the controller-transmitter 120 and to deliver one or more electrical pulses to the heart 102 based on the received acoustic energy. The various receiver-stimulators shown in and described in detail with reference to Figures 2-10 can include some features that are at least generally similar in structure and function, or identical in structure and function, to one another. In some embodiments, aspects of the various embodiments can be combined. In some embodiments, similar or identical elements are identified by reference numbers having the same final two digits. For example, elements 210 and 310 can include some features that are at least generally similar in structure and function, or identical in structure and function, to one another.

Figure 2 is a side view of a pair of receiver-stimulators 210 (identified individually as a first receiver-stimulator 210a and a second receiver-stimulator 210b) secured to a septal wall SW of a heart of a patient and within a left ventricle LV of the heart in accordance with embodiments of the present technology. The septal wall SW separates the left ventricle LV of the heart from a right ventricle RV. In the illustrated embodiment, the receiver-stimulators 210 are identical and each include a body 212 and an anchor 214 extending from the body 212. In some embodiments, the bodies 212 each have a generally cylindrical shape while, in other embodiments, the bodies 212 can have other shapes (e.g., including a rectangular, square, polygonal, rectilinear, irregular, and/or other cross-sectional shape). The anchors 214 can each extend into the septal wall SW to secure the receiver-stimulators 210 thereto, and can each carry one or more electrodes 216, such as a pair of bipolar pacing electrodes. In some embodiments, the anchors 214 each have a corkscrew-like shape. As described in detail above with reference to Figure 1, the receiver-stimulators 210 can each include circuitry positioned within the bodies 212 and configured to (i) receive energy (e.g., directed acoustic energy) from the controller-transmitter 120 (Figure 1), (ii) convert the energy to electrical energy, and (iii) output the electrical energy via the electrodes 216 to simulate tissue of the septal wall SW adjacent the electrodes 216.

In some embodiments, the receiver-stimulators 210 can include some features that are at least generally similar in structure and function, or identical in structure and function, to those of the receiver-stimulators disclosed in any of (i) U.S. Patent No. 7,848,815, filed September 4, 2009, and titled "IMPLANTABLE TRANSDUCER DEVICES"; (ii) U.S. Patent No. 7,606,621, filed December 21, 2005, and titled "IMPLANTABLE TRANSDUCER DEVICES"; (iii) U.S. Patent No. 7,610,092, filed December 21, 2005, and titled "LEADLESS TISSUE STIMULATION SYSTEMS AND METHODS"; (iv) U.S. Patent No. 9,616,237, filed September 30, 2013, and titled "SYSTEMS, DEVICES, AND METHODS FOR SELECTIVELY LOCATING IMPLANTABLE DEVICES"; (v) U.S. Patent No. 9,343,654, filed October 15, 2015, and titled "METHOD OF MANUFACTURING IMPLANTABLE WIRELESS ACOUSTIC STIMULATORS WITH HIGH ENERGY CONVERSION EFFICIENCIES"; and/or (vi) U.S. Patent No. 9,283,392, filed September 24, 2010, and titled "TEMPORARY ELECTRODE CONNECTION FOR WIRELESS PACING SYSTEMS,".

Various conductive cardiac structures can extend through the septal wall SW, such as the bundle of His, the left bundle branch, the right bundle branch, and so on. In some embodiments, one of the receiver-stimulators 210 (e.g., the first receiver-stimulator 210a) can be positioned near the bundle of His and another one of the receiver-stimulators 210 (e.g., the second receiver-stimulator 210b) can be positioned below the first receiver-stimulator 210a near the left bundle branch. Alternatively, additional ones of the receiver-stimulators 210 (not shown) can be positioned in the region. In some aspects of the present technology, the receiver-stimulators 210 can be relatively smaller and have a lower pacing output than some known receiver-stimulators because the pacing stimulation is delivered in close proximity to targeted conduction structures (e.g., the bundle of His, the left bundle branch) and therefore does not require as much energy as compared to other locations in the heart.

In some embodiments, each of the receiver-stimulators 210 can have a distinct operating code and can be uniquely addressed by a controller-transmitter (e.g., the controller-transmitter 120 of Figure 1). Accordingly, the receiver-stimulators 210 can operate to pace the septal wall SW of the left ventricle L V simultaneously, one at a time, and/or in a staggered manner (e.g., separated by a programmable delay). For example, the pacing output for the two receiver-stimulators 210 shown in Figure 2 can be programmed for (i) a single pacing output only (e.g., by the first receiver-stimulator 210a positioned near the bundle of His), (ii) a simultaneous pacing output by both of the receiver-stimulators 210, and/or (iii) a first pacing output by the first receiver-stimulator 210a (e.g., positioned nearest the bundle of **His)** that is followed-after a programmable time delay-by a second pacing output by the second receiver-stimulator 210b (e.g., positioned nearest the left bundle branch). Although two of the receiver-stimulators 210 are shown **in** Figure 2, any number of unique receiver-stimulators can be used together as a multi-receiver stimulator system. In some embodiments, the pacing output from the receiver-stimulators 210 includes neuromodulation pulses for stimulating the nerve structure within the septal wall SW. In some embodiments, the neuromodulation pulses have a pulse width of 100 microseconds and/or an amplitude of 1-1.5 volts.

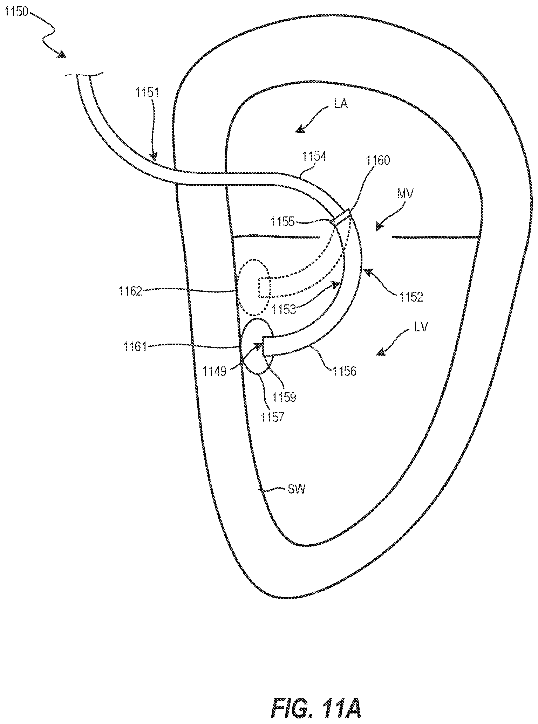

In some embodiments, the receiver-stimulators 210 are delivered to the septal wall SW via a delivery catheter inserted through a curved sheath. For example, the receiver-stimulators 210 can be delivered to the septal wall SW using any of the delivery systems described in detail below with reference to Figures 11A-15K and, for example, more particularly Figures 11A-12. In some embodiments, the anchors 214 for each of the receiver-stimulators 210 are secured in the septal wall SW by rotating an associated delivery catheter to "screw" the anchor 214 into the septal wall SW. In other embodiments, the anchors 214 for each of the receiver-stimulators 210 are secured in the septal wall SW via a push-to-anchor method or via a pull-back-to-deploy and push-to-anchor method.

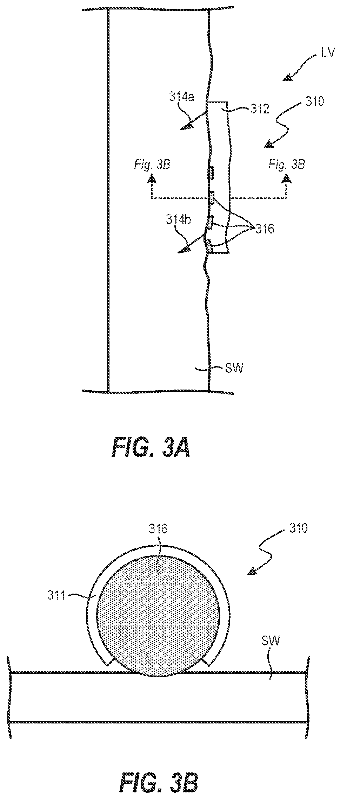

Figures 3A and 3B are a side view and a transverse cross-sectional view, respectively, of a receiver-stimulator 310 secured to the septal wall SW within the left ventricle LV in accordance with embodiments of the present technology. Referring to Figure 3A, in the illustrated embodiment the receiver-stimulator 310 includes a body 312 having a plurality of electrodes 316 attached thereto and/or integrally formed therein. The receiver-stimulator 310 can further include multiple anchors 314 (e.g., identified individually as a proximal anchor 314a and a distal anchor 314b) that can be inserted at least partially into the septal wall SW to secure the electrodes 316 in contract with the septal wall SW. In the illustrated embodiment, the anchors 314 do not include electrodes thereon while, in other embodiments, the anchors 314 can include electrodes thereon.

The receiver-stimulator 310 can include circuitry configured to (i) receive energy (e.g., directed acoustic energy) from the controller-transmitter 120 (Figure 1), (ii) convert the energy to electrical energy, and (iii) output the electrical energy via the electrodes 316 to simulate the tissue of the septal wall SW adjacent the electrodes 316. In some embodiments, the electrodes 316 form a quadripolar electrode array (e.g., a single linear quadripolar electrode array) that contacts the septal wall SW. Referring to Figure 3B, in some embodiments the receiver-stimulator 310 has a circular cross-sectional shape and is formed of an electrode material. The receiver-stimulator 310 can include a masking or coating 311 over the electrode material that includes openings that define the electrodes 316. The coating 311 can be nonconductive (e.g., formed of an electrically-insulative material) such that the electrodes 316 are only exposed adjacent the septal wall SW to provide a direct stimulation path into the septal wall SW. For example, the coating 311 can comprise a polymer (e.g., parylene) and can be positioned around about 270 degrees of a circumference of the electrodes 316. In some aspects of the present technology, the coating 311 can help ensure that most of the pacing electrical energy is delivered into the septal wall SW where the electrodes 316 contact the septal wall SW.

In some embodiments, the receiver-stimulator 310 has programmable electrode configurations to, for example, provide several combinations of pacing vectors along the septal wall SW. For example, the electrodes 316 can be spatially programmable in the same or a similar manner as the electrodes 216 described in detail above with reference to Figure 2. Many programming combinations are possible and, in some embodiments, timing delays can be programmed as well. The receiver-stimulator 310 can include an application-specific integrated circuit (ASIC) configured to enable this programmability.

In some embodiments, the receiver-stimulator 310 is delivered to the septal wall SW via a delivery catheter such that the distal anchor 314b is inserted in the septal wall SW first. Then, with a slight lateral move from the delivery catheter, the catheter can insert the proximal anchor 314a into the septal wall SW to secure the receiver-stimulator 310 in position. In some aspects of the present technology, this anchoring technique and the arrangement of the electrodes 316 enables the receiver-stimulator 310 to be positioned in a parallel orientation to the septal wall SW rather than the perpendicular orientation shown in Figure 3A.

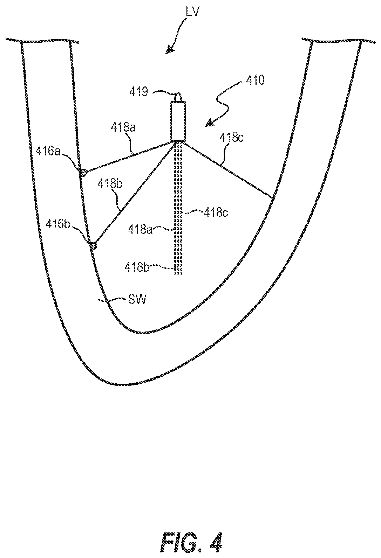

Figure 4 is a side view of a receiver-stimulator 410 positioned in the left ventricle LV in accordance with embodiments of the present technology. In the illustrated embodiment, the receiver-stimulator includes a body 412 and a plurality of elongate legs 418 (identified individually as first through third legs 418a-c, respectively) extending from the body 412. The receiver-stimulator 410 can further includes multiple electrodes 416 (including an individually identified first electrode 416a and a second electrode 416b) carried by the legs 418. For example, in the illustrated embodiment the first leg 418a carries the first electrode 416a and the second leg 418b carries the second electrode 416b. The electrodes 416 can be unipolar or bipolar electrode sets. The legs 418 can be expandable from a compressed delivery position (shown in phantom lines in Figure 4) to an expanded deployed position shown in Figure 4 in which the legs 418 form a tripod. In some embodiments, the receiver-stimulator 410 includes a spring mechanism 419 that is actuatable (e.g., that can be pushed and/or pulled via an associated delivery system) to allow the legs 418 to expand to the deployed position. In some embodiments, the legs 418 can be deployed from the compressed delivery position to the expanded deployed position via other actuation mechanisms of a delivery catheter used to deliver the receiver-stimulator 410 to the left ventricle LV.

In the deployed position, distal portions of the legs 418 can contact the walls of the left ventricle LV to (i) secure the receiver-stimulator 410 in position within the left ventricle LV and (ii) contact the electrodes 416 with the septal wall SW. More specifically, the first and second legs 418a-b (e.g., active electrode legs) can drive the electrodes 416 into contact with the septal wall SW, while the third leg 418 (e.g., a stabilization leg) contacts the wall opposite the septal wall SW (e.g., a lateral free wall of the left ventricle LV) to provide stabilization. The legs 418 can be secured to the respective walls of the left ventricle LV by an outward spring force and/or by one or more anchoring mechanisms (not shown). The electrodes 416 can have programmable electrode configurations as described in detail above.

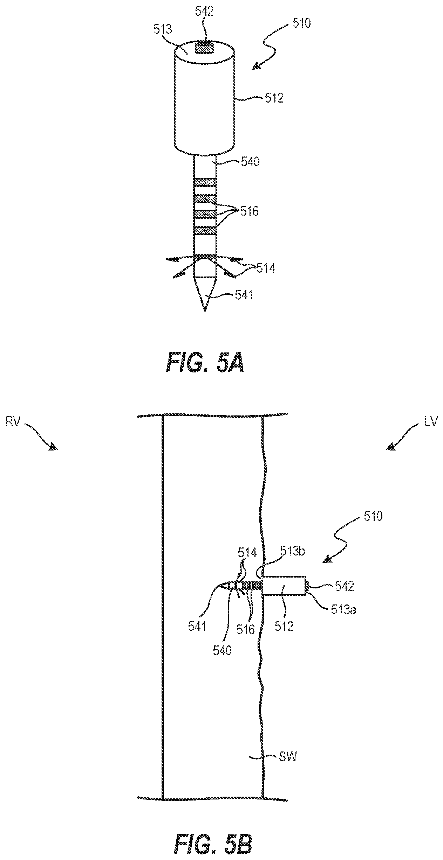

Figure 5A is an isometric view of a receiver-stimulator 510, and Figure 5B is a side view of the receiver-stimulator 510 secured to the septal wall SW within the left ventricle LV, in accordance with embodiments of the present technology. Referring to Figures 5A and 5B, in the illustrated embodiment the receiver-stimulator 510 includes a body 512 and an elongate member or needle 540 extending from the body 512. The needle 540 can have a pointed tip 541 configured to penetrate the septal wall SW. In the illustrated embodiment, the needle 540 carries one or more electrodes 516 and one or more anchors 514. In some embodiments, the body 512 can be positioned in either the left ventricle LV or the right ventricle RV, and the needle 540 can penetrate the septal wall SW. That is, the receiver-stimulator 510 can be delivered through either the right ventricle RV or the left ventricle LV. The anchors 514 can be barbs, tines, hooks, and/or other members that extend away from the needle 540 and that are configured (e.g., shaped and sized) to secure the needle 540 within the septal wall SW. In some embodiments, the body 512 includes a proximal surface 513a spaced apart from the septal wall SW and opposite a distal surface 513b adjacent the septal wall SW. The needle 540 can extend from the distal surface 513b, and the proximal surface 513a can carry a non-contacting electrode 542. In some embodiments, the electrodes 516 on the needle 540 are pacing cathode electrodes and the non-contacting electrode 542 is an anode that can electrically communicate with one or more of the pacing cathode electrodes 516. In some embodiments, the needle 540 can have a variable length that enables more or fewer of the electrodes 516 to be selectively positioned within the septal wall SW to provide a desired stimulation pattern. In some embodiments, energy can be selectively applied to the electrodes 516 to provide stimulation at different depths within the septal wall SW.

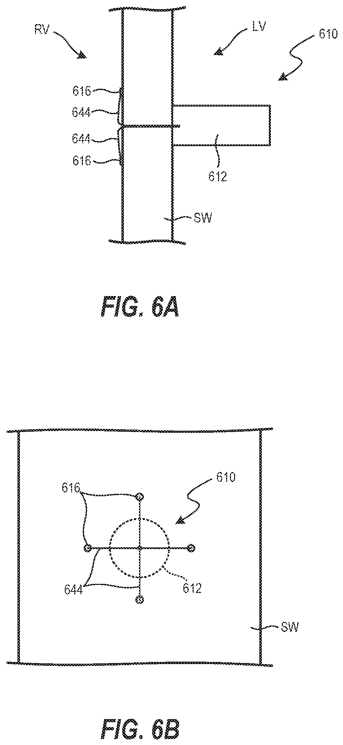

Figure 6A is a side view of a receiver-stimulator 610, and Figure 6B is a front view (e.g., proximally facing) from inside the right ventricle RV of the receiver-stimulator 610 secured to the septal wall SW, in accordance with embodiments of the present technology. In the illustrated embodiment, the receiver-stimulator 610 includes a body 612 and a transseptal anchoring system comprising a plurality of tines 644 (e.g., bendable members, anchors, securement members) each carrying a corresponding one of a plurality of electrodes 616 at a distal portion thereof. The tines 644 can extend through the septal wall SW from the body 612 in the left ventricle LV and into the right ventricle RV. In the right ventricle RV, the tines 644 can bend parallel to the septal wall SW and/or back toward the septal wall SW to (i) secure the electrodes 616 in contact with a surface of the septal wall SW in the right ventricle RV and (ii) secure the receiver-stimulator 610 relative to the septal wall SW. In other embodiments, the body 612 of the receiver-stimulator 610 can be positioned in the right ventricle RV and the tines 644 can extend through the septal wall SW into the left ventricle LV to secure the electrodes 616 in contact with a surface of the septal wall SW in the left ventricle LV.

Referring to Figure 6B, in some embodiments the receiver-stimulator 610 includes four of the tines 644 that are configured to be deployed at 90 degrees relative to one another. In other embodiments, the receiver-stimulator 610 can include more or fewer of the tines 644, and/or individual ones of the tines 644 can include more or fewer of the electrodes 616. The electrodes 616 can be programmed to provide a desired pacing pattern to the septal wall SW. For example, the pacing output and timing of each of the electrodes 616 can be individually controllable.

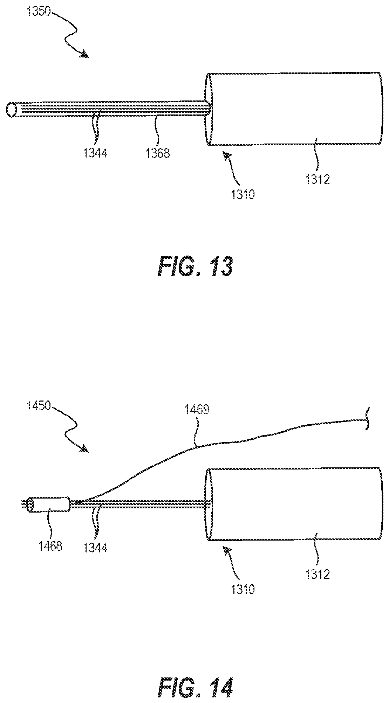

In some embodiments, the receiver-stimulator 610 can be delivered through either the right ventricle RV or the left ventricle LV in a compressed configuration in which the tines 644 are oriented generally parallel to one another and a common axis. In some embodiments, the tines 644 are formed of a shape-memory material or otherwise configured to deflect outwardly to the deployed configuration shown in Figures 6A and 6B from the compressed delivery configuration. More specifically, in the deployed configuration at least a portion (e.g., a distal portion) of each of the tines 644 can be configured to deflect away from the common axis and toward the surface of the septal wall SW. In some embodiments, the receiver-stimulator 610 can be delivered using any of the delivery systems described in detail below with reference to Figures 11A-15K and, for example, more particularly Figures 13 and 14.

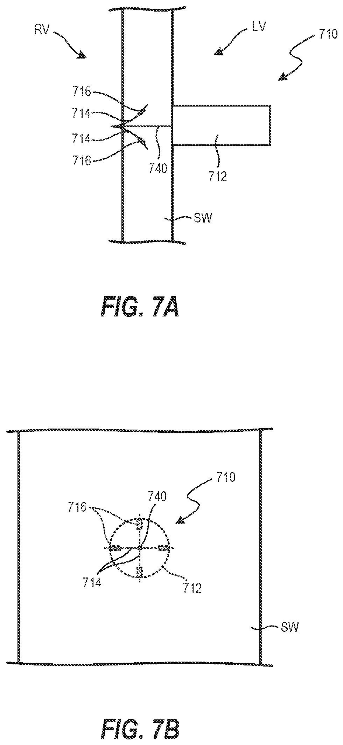

Figure 7A is a side view of a receiver-stimulator 710, and Figure 7B is a front view (e.g., proximally facing) from inside the right ventricle RV of the receiver-stimulator 710 secured to the septal wall SW, in accordance with embodiments of the present technology. In the illustrated embodiment, the receiver-stimulator 710 includes a body 712 and an anchoring system comprising a needle 740 extending from the body 712 and a plurality of tines or anchors 714 extending from the needle 740. The anchors 714 can each carry a corresponding one of a plurality of electrodes 716. In some embodiments, the anchors 714 extend into the septal wall SW to (i) secure the electrodes 716 in contact with and within the septal wall SW and (ii) secure the receiver-stimulator 710 relative to the septal wall SW. In the illustrated embodiment, the body 712 is positioned within the left ventricle LV and a portion of the needle 740 and the anchors 714 extends entirely through the septal wall SW into the right ventricle RV. In other embodiments, the receiver-stimulator 710 can be positioned in an opposite manner with the body 712 in the right ventricle RV, and/or the needle 740 and the anchors 714 need not cross the entirety of the septal wall SW (e.g., can be positioned entirely within the septal wall SW).

Referring to Figure 7B, in some embodiments the receiver-stimulator 710 includes four of the anchors 714 that are configured to be deployed at 90 degrees relative to one another. In other embodiments, the receiver-stimulator 710 can include more or fewer of the anchors 714, and/or individual ones of the anchors 714 can include more or fewer of the electrodes 716. The electrodes 716 can be programmed to provide a desired pacing pattern output to the septal wall SW. For example, the pacing output and timing of each of the electrodes 716 can be individually controllable.

In some embodiments, the receiver-stimulator 710 can be delivered through either the right ventricle RV or the left ventricle LV in a compressed configuration in which the anchors 714 are oriented generally parallel to one another. In some embodiments, the anchors 714 are formed of a shape-memory material or otherwise configured to deflect outwardly to the deployed configuration shown in Figures 7A and 7B from the compressed delivery configuration. In some embodiments, the receiver-stimulator 710 can be delivered using any of the delivery systems described in detail below with reference to Figures 11A-15K and, for example, more particularly Figures 13 and 14.

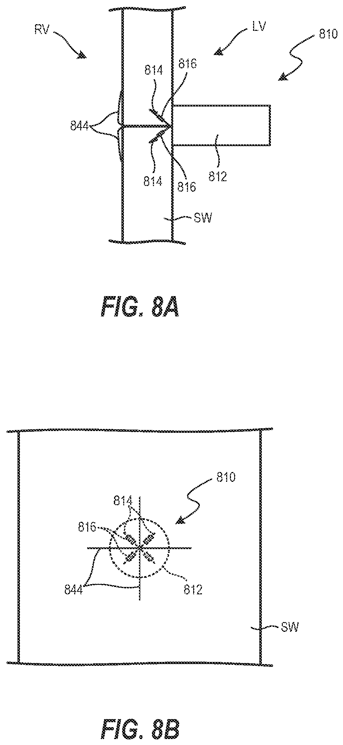

Figure 8A is a side view of a receiver-stimulator 810, and Figure 8B is a front view (e.g., proximally facing) from inside the right ventricle RV of the receiver-stimulator 810 secured to the septal wall SW, in accordance with embodiments of the present technology. In the illustrated embodiment, the receiver-stimulator 810 includes a body 812 and an anchoring system comprising a plurality of tines 844 and anchors 814 extending from the body 812. The anchors 814 can each carry a corresponding one of a plurality of electrodes 816. In other embodiments, the tines 844 (e.g., distal portions of the tines 844) can alternatively or additionally carry corresponding ones of the electrodes 816.

The tines 844 can extend through the septal wall SW from the body 812 in the left ventricle LV and into the right ventricle RV. In the right ventricle RV, the tines 844 can bend parallel to the septal wall SW and/or back toward the septal wall SW to help secure the receiver-stimulator 810 relative to the septal wall SW. The anchors 814 can extend into the septal wall SW to (i) secure the electrodes 816 in contact with and within the septal wall SW and (ii) help secure the receiver-stimulator 810 relative to the septal wall SW. In other embodiments, the receiver-stimulator 810 can be positioned in an opposite manner with the body 812 in the right ventricle RV such that the tines 844 extend through the septal wall SW from the right ventricle RV and into the left ventricle LV.

Referring to Figure 8B, in some embodiments the receiver-stimulator 810 includes four of the anchors 814 that are configured to be deployed at 90 degrees relative to one another, and four of the tines 844 that are configured to be deployed at 90 degrees relative to one another. The anchors 814 can further be interspersed/interleaved between the tines 844 (e.g., offset by 45 degrees relative to one another). In other embodiments, the receiver-stimulator 810 can include more or fewer of the anchors 814 and/or the tines 844, and/or the anchors 814 and the tines 844 can be positioned differently relative to one another. In some embodiments, the receiver-stimulator 810 can be delivered through either the right ventricle RV or the left ventricle LV in a compressed configuration in which the anchors 814 and the tines 844 are oriented generally parallel to one another and a common axis. In some embodiments, the anchors 814 and the tines 844 are formed of a shape-memory material or otherwise configured to deflect outwardly (e.g., away from the common axis) from the compressed delivery configuration to the deployed configuration shown in Figures 8A and 8B. In some embodiments, the receiver-stimulator 810 can be delivered using any of the delivery systems described in detail below with reference to Figures 11A-15K and, for example, more particularly Figures 13 and 14.

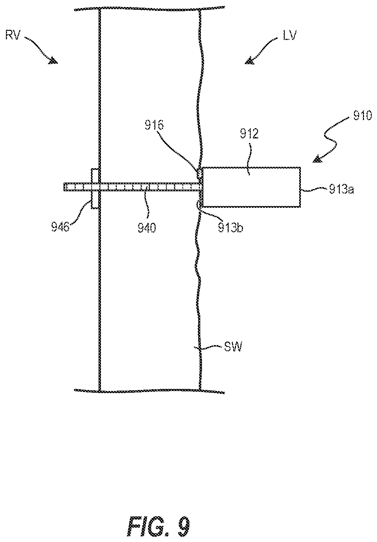

Figure 9 is a side view of a receiver-stimulator 910 secured to the septal wall SW in accordance with embodiments of the present technology. In the illustrated embodiment, the receiver-stimulator 910 includes a body 912 and an elongate member or needle 940 extending from the body 912. The body 912 includes a proximal surface 913a spaced apart from the septal wall SW and opposite a distal surface 913b adjacent the septal wall SW. The needle 940 can extend from the distal surface 913b and the receiver-stimulator 910 can include an electrode mounted to the distal surface 913b. In some embodiments, the electrode 916 is eccentrically mounted to the distal surface 913b such that rotation of the receiver-stimulator 910 changes the position of the electrode 916 along the septal wall SW (e.g., to provide for adjustment after anchoring). That is, for example, the body 912 can include a longitudinal axis extending perpendicular to the distal surface 913b and coincident with the needle 940, and the electrode 916 can be positioned off (e.g., away from) the longitudinal axis on the distal surface 913b. In some embodiments, the electrode 916 is a cathode pacing electrode. In some embodiments, the electrode 916 is isolated from the needle 940 and/or the anchor member 946 to, for example, provide for minimal fibrotic cap and low pacing thresholds.

In some embodiments, the body 912 is positioned within the left ventricle LV and the needle 940 extends through the septal wall SW from the left ventricle LV into the right ventricle RV. In the illustrated embodiment, the receiver-stimulator 910 further includes an anchor member 946, such as a bushing, positioned in the right ventricle RV and secured to the needle 940 (e.g., a distal portion of the needle 940). In some embodiments, the needle 940 can be threaded and the anchor member 946 can include corresponding threads such that the anchor member 946 can be screwed onto the needle 940. The anchor member 946 can (i) secure the electrode 916 in contact with the surface of the septal wall SW (e.g., by pulling the electrode 916 toward the septal wall SW) and (ii) secure the receiver-stimulator 910 relative to the septal wall SW. Accordingly, in some aspects of the present technology the receiver-stimulator 910 is securely attached to the septal wall SW via compression-rather than extension-of the septal wall SW. In other embodiments, the receiver-stimulator 910 can be positioned in an opposite manner with the body 912 in the right ventricle RV and the anchor member 946 in the left ventricle LV.

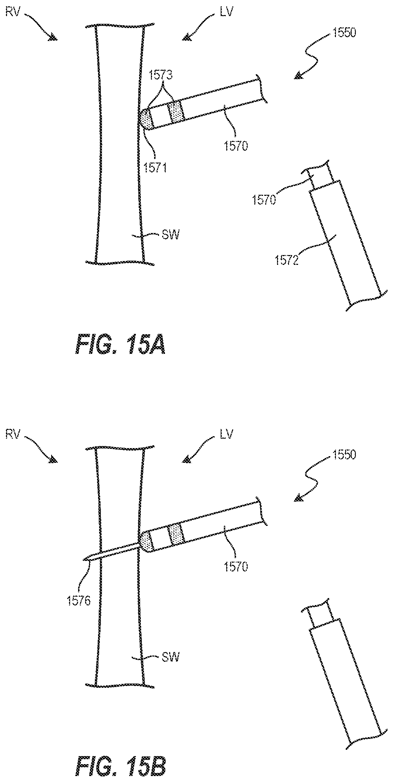

In some embodiments, the body 912 and needle 940 are delivered into the left ventricle LV and through the septal wall SW, and then the anchor member 946 is delivered into the right ventricle RV and secured to the needle 940. In some embodiments, the receiver-stimulator 910 can be delivered using any of the delivery systems described in detail below with reference to Figures 11A-15K and, for example, more particularly Figures 15A-15K.

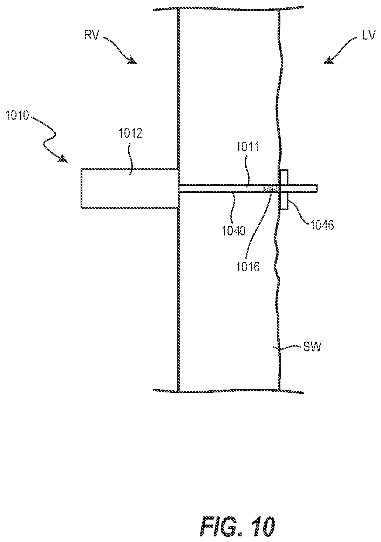

Figure 10 is a side view of a receiver-stimulator 1010 secured to the septal wall SW of the left ventricle LV in accordance with embodiments of the present technology. In the illustrated embodiment, the receiver-stimulator 1010 includes several features generally similar to those of the receiver-stimulator 910 described in detail above with reference to Figure 9, such as a body 1012, a needle 1040, and an anchor member 1046. However, in the illustrated embodiment the body 1012 is positioned in the right ventricle RV, the anchor member 1046 is positioned in the left ventricle LV, and the needle 1040 includes an electrode 1016 (e.g., rather than the body 1012 including a separate electrode mounted thereto). The electrode 1016 can be positioned along the needle 1040 such that it is positioned close to the left ventricle LV (e.g., at and/or proximate the surface of the septal wall SW in the left ventricle LV) when the receiver-stimulator 1010 is implanted as shown in Figure 10. In some embodiments, the needle 1040 can comprise an electrode material and can be coated with an insulative material 1011 with an opening that defines the electrode 1016.

### III. Selected Embodiments of Delivery Systems and Components

Figures 11A-15K illustrate various delivery systems for implanting a one or more receiver-stimulators to stimulate, for example, the left ventricular septal wall in accordance with embodiments of the present technology. The delivery systems can be used to deliver one or more of the receiver-stimulators described in detail above with reference to Figures 2-10. The various receiver-stimulators shown in and described in detail with reference to Figures 11A-15K can include some features that are at least generally similar in structure and function, or identical in structure and function, to one another. In some embodiments, aspects of the various embodiments can be combined. In some embodiments, similar or identical elements are identified by reference numbers having the same final two digits. For example, elements 1150 and 1250 can include some features that are at least generally similar in structure and function, or identical in structure and function, to one another.

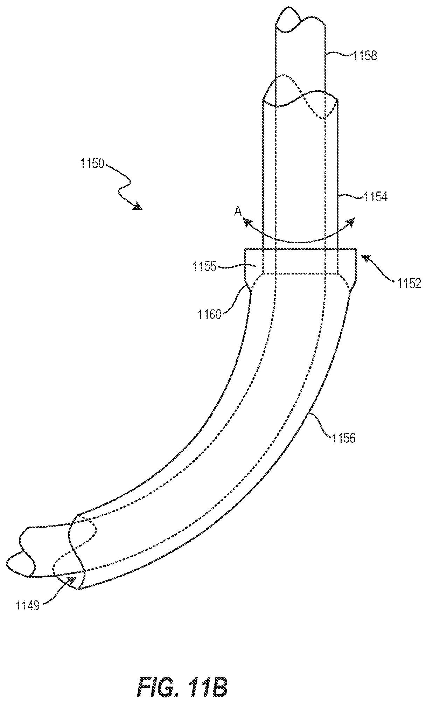

Figure 11A is a side view of a distal portion of a delivery system 1150 configured to implant a receiver-stimulator within the heart of a patient in accordance with embodiments of the present technology. Figure 11B is an enlarged side view of the distal portion of the delivery system 1150 in accordance with embodiments of the present technology. Referring to Figures 11A and 11B, in the illustrated embodiment the delivery system 1150 includes an elongate sheath 1152 (which can also be referred to a first catheter or a first elongate member) defining a lumen 1149 and having a proximal segment or portion 1154 rotatably coupled to a distal segment or portion 1156 via a rotatable coupling 1155. A delivery catheter 1158 (obscured in Figure 11A; which can also be referred to a second elongate sheath or a second elongate member) can be advanceable through the lumen 1149 of the sheath 1152. In some embodiments, a receiver-stimulator, such as one or more of receiver-stimulators described in detail above with reference to Figures 2-10, can be coupled to the delivery catheter 1158 and/or advanced through the delivery catheter 1158 for implantation at the septal wall SW within the left ventricle LV of the heart. For example, a receiver-stimulator can be clamped to a distal portion of the delivery catheter 1158.

The rotatable coupling 1155 can be a bearing, gland, or other member that allows the distal region 1156 and the proximal region 1154 of the sheath 1152 to rotate relative to one another. In some embodiments, the proximal region 1154 of the sheath 1152 is secured to the distal region 1156 via an interference fit, a snap-fit arrangement, and/or another suitable connection at the rotatable coupling 1155. In some aspects of the present technology, the rotatable coupling 1155 can inhibit or even prevent the distal region 1156 of the sheath 1152 from separating from the proximal region 1154 during withdrawal of the sheath 1152 under tension. In some embodiments, the rotatable coupling 1155 is configured to permit the distal region 1156 to rotate relative to the proximal region 1154 of the sheath 1152 (e.g., as indicated by arrow A in Figure 11B) by more than about 50 degrees, more than about 90 degrees, more than about 180 degrees, and/or more than about 270 degrees about a longitudinal axis of the sheath 1152. In some embodiments, the rotatable coupling 1155 can include an outer surface 1160 that is at least partially chamfered or angled to facilitate smooth advancement through an introducer and/or the vasculature of the patient.

Referring to Figure 11A, in some embodiments the sheath 1152 is configured to be advanced transeptally through the septal wall SW, into a left atrium LA of the patient, through/past a mitral valve MV of the patient, and into the left ventricle LV. In the illustrated embodiment, the proximal region 1154 of the sheath 1152 defines a proximal bend 1151 and the distal region 1156 of the sheath 1152 defines a distal bend 1153. In some embodiments, the proximal bend 1151 has a radius of curvature (e.g., a turning radius) that is larger than a radius of curvature of the distal bend 1153. In some embodiments, a balloon 1157 (shown in crosssection in Figure 11A) can be coupled to a distal terminus 1159 of the sheath 1152. In some aspects of the present technology, the distal bend 1153 is shaped and sized to help position the balloon 1157 along the septal wall SW within the left ventricle LV, while the proximal bend 1151 facilitates entry of the delivery system 1150 into the left atrium LA. In some embodiments, the delivery system 1150 can include other components (not shown) in addition to the rotatable sheath 1152 and the delivery catheter 1158 such as, for example, a transseptal needle, transseptal dilator, transseptal sheath, and/or the like.

Referring again to Figures 11A and 11B, during a delivery procedure, a transseptal puncture can be performed in the superior and posterior-mid aspect of the fossa ovalis in most patients. The delivery system 1150 can then be advanced through the puncture and across the septal wall between the right atrium and left atrium LA at a distance from an annulus of the mitral valve MV of, for example, between about 3.5-4.0 centimeters. In some embodiments, the sheath 1152 can then be rotated by actuating the distal region 1156 of the sheath (e.g., via a cable and knob on a proximal handle of the sheath 1152) and then torqueing the delivery catheter 1158 while the receiver-stimulator is still secured thereto. That is, in some embodiments the delivery catheter 1158 can be torqued to rotate the distal region 1156 of the sheath 1152 while the receiver-stimulator is not released from the delivery catheter 1158 and not protruding distally outside of the balloon 1157. Such rotation can allow the balloon 1157 to be positioned at different target locations along the septal wall SW, and thus for the receiver-stimulator-or multiple different receiver-stimulators-to be delivered to and implanted at one of the different locations. For example, in Figure 11A the delivery system 1150 is positioned to implant the receiver-stimulator at a first target location 1161 along the septal wall SW within the left ventricle LV, but can be rotated (as shown in phantom lines) to implant the receiver-stimulator at a second target location 1162 along the septal wall SW and/or other target locations. In some embodiments, multiple delivery catheters can be inserted through the sheath 1152 to implant multiple receiver-stimulators at different locations along the sepal wall SW.

In other embodiments, the delivery system 1150 can be advanced intravascularly into a right ventricle of the patient to, for example, facilitate delivery of a receiver-stimulator to the septal wall SW within the right ventricle. In such embodiments, the distal bend 1153 can be similarly shaped and sized to help position the balloon 1157 along the septal wall SW within the right ventricle, and the sheath 1152 can be rotated to position the balloon 1157 at different target sites along the septal wall SW.

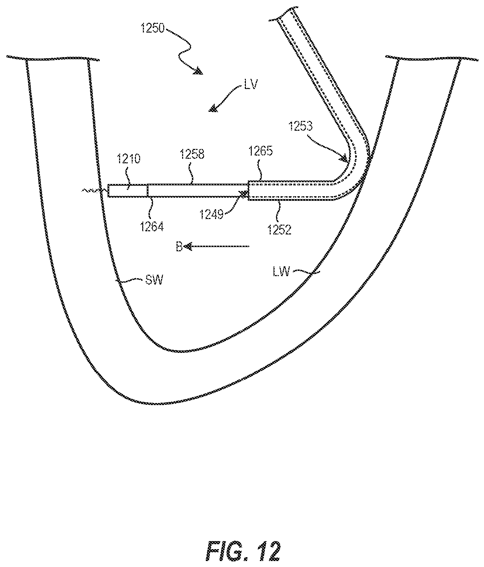

Figure 12 is a side view of a distal portion of a delivery system 1250 configured to implant a receiver-stimulator 1210 within the heart of a patient in accordance with embodiments of the present technology. In the illustrated embodiment, the delivery system 1250 includes an elongate sheath 1252 defining a lumen 1249, and a delivery catheter 1258 that is advanceable through the lumen 1249 of the sheath 1252. In some embodiments, the receiver-stimulator 1210 (e.g., which can be similar or identical to the receiver-stimulator 210 described in detail with reference to Figure 2) can be coupled to a distal portion 1264 of the delivery catheter 1258 and/or advanced through the delivery catheter 1258 for implantation at the septal wall SW within the left ventricle LV of the heart.

In the illustrated embodiment, the sheath 1252 has a shape including a distal bend 1253 and a generally straight distal portion 1265 distal of the distal bend 1253. During a delivery procedure, the sheath 1252 can be advanced over the delivery catheter 1258 and/or the delivery catheter 1258 can be advanced through the sheath 1252 such that (i) the distal bend 1253 contacts a posterior or lateral wall LW within the left ventricle opposite the septal wall SW and (ii) the distal portion 1265 faces (e.g., generally orthogonally faces) the septal wall SW. Accordingly, in some aspects of the present technology the sheath 1252 can baffle against the lateral wall LW to apply to apply a forward anchoring force to the receiver-stimulator 1210 (e.g., in a direction indicated by the arrow B toward the septal wall SW) during implantation of the receiver-stimulator 1210 using the delivery catheter 1258. In some embodiments, the generally straight distal portion 1265 can have a controllably variable length to, for example, allow for changes in a minimum bend radius of the distal bend 1253 to facilitate positioning of the delivery system 1250 in the left ventricle LV.

In some aspects of the present technology, much of the challenge in reaching target implantation locations along the septal wall SW within the left ventricle LV is the relative inflexibility of the delivery catheter 1258. Accordingly, in some embodiments a length of the receiver-stimulator 1210 and/or an associated mechanism for detaching the receiver-stimulator 1210 from the delivery catheter 1258 can be decreased to decrease a corresponding length of a relatively stiff section of the delivery catheter 1258 to further improve the flexibility of the delivery system 1250 and the ability to deliver the receiver-stimulator 1210 to a desired target location along the septal wall SW. Similarly, in some embodiments the delivery catheter 1258, the receiver-stimulator 1210, and/or an associated detachment mechanism can include one or more hinges, pivot points, and/or the like to reduce the stiffness of the delivery system 1250. For example, the receiver-stimulator 1210 can be pivotably coupled to the delivery catheter 1258 to improve flexibility.

Figure 13 is a side view of a portion of a delivery system 1350 configured to implant a receiver-stimulator 1310 within the heart of a patient in accordance with embodiments of the present technology. In some embodiments, the receiver-stimulator 1310 can be similar or identical to any of the receiver-stimulators described in detail above with reference to Figures 6A-8B. For example, in the illustrated embodiment the receiver-stimulator 1310 includes a body 1312 and a plurality of tines 1344 than can carry one more stimulation electrodes (not shown).

The receiver-stimulator 1310 is in a compressed delivery configuration in Figure 13 in which the tines 1344 are oriented generally parallel to one another. More specifically, the delivery system 1350 can include a sleeve 1368 (shown as transparent in Figure 13 for clarity) that at least partially surrounds the tines 1344 during delivery of the receiver-stimulator 1310. The sleeve 1368 can maintain the tines 1344 in the compressed delivery configuration during delivery and/or implantation and can be removed after delivery of the receiver-stimulator 1310 to a target implant location to allow the tines 1344 to expand and secure the receiver-stimulator 1310 to the target implant location. In some embodiments, for example, the sleeve 1368 is formed of a biodegradable material (e.g., a rapidly biodegradable material) that degrades after implantation allowing the tines 1344 to expand, such as within the ventricle of a patient as described in detail above with reference to Figures 6A-8B. In other embodiments, the sleeve 1368 can include perforations and/or the delivery system 1350 can include a suture or other device for slitting the sleeve 1368 to remove the sleeve 1368 from around the tines 1344 to permit the tines 1344 to expand.

Figure 14 is a side view of a portion of a delivery system 1450 configured to implant the receiver-stimulator 1310 of Figure 13 within the heart of a patient in accordance with embodiments of the present technology. The receiver-stimulator 1310 is in the compressed delivery configuration in Figure 13 in which the tines 1344 are oriented generally parallel to one another. More specifically, the delivery system 1450 can include a sleeve 1468 that at least partially surrounds the tines 1344 during delivery of the receiver-stimulator 1310. The sleeve 1468 can maintain the tines 1344 in the compressed delivery configuration during delivery and/or implantation and can be removed after delivery of the receiver-stimulator 1310 to a target implant location to allow the tines 1344 to expand and secure the receiver-stimulator 1310 to the target implant location. In the illustrated embodiment, for example, the sleeve 1468 is coupled to a pull string or pull wire 1469 that can, for example, extend proximally to a handle of the delivery system 1450 and/or proximally outside of the patient. When the receiver-stimulator 1310 is positioned at the target location, the pull wire 1469 can be pulled and/or pushed to move the sleeve 1468 distally off of the tines 1344 or proximally toward the body 1312 to permit the tines 1344 to expand.

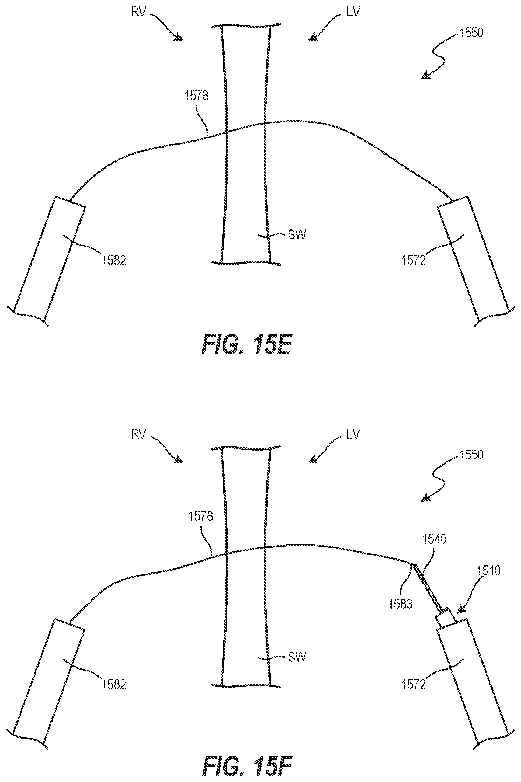

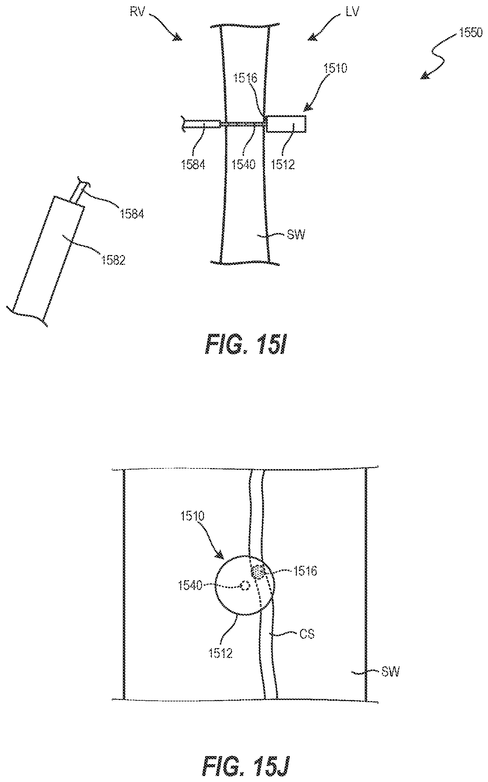

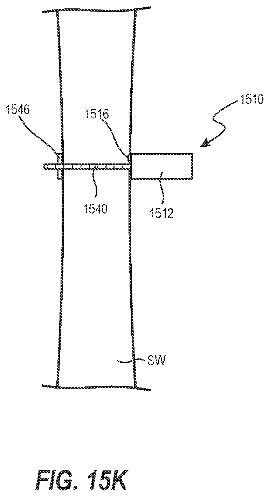

Figures 15A-15I and 15K are side views of a distal portion of a delivery system 1550 during different stages of a procedure to implant a receiver-stimulator 1510 (Figures 15F-15K) within the septal wall SW of a heart of a patient in accordance with embodiments of the present technology. Figure 15J is a rear view (e.g., distally-facing) from within the left ventricle LV of the receiver-stimulator 1510 implanted at the septal wall SW in accordance with embodiments of the present technology. In some embodiments, the receiver-stimulator 1510 can be similar or identical to any of the receiver-stimulators described in detail above with reference to Figures 9 and 10. For example, as best shown in Figure 15K, the receiver-stimulator 1510 can include a body 1512 including an electrode 1516 and having a needle 1540 extending therefrom and configured to penetrate the septal wall SW. An anchor member 1546 can be coupled to the needle 1540 to secure the receiver-stimulator 1510 and the electrode 1516 against the septal wall SW.

Figure 15A illustrates the delivery system 1550 after advancement of a first catheter 1570 (e.g., a mapping catheter) through a first sheath 1572, into the left ventricle LV, and toward the septal wall SW. The first sheath 1572 can be positioned at least partially within the left ventricle LV or can be positioned proximally within the vasculature of the patient. In some embodiments, the first catheter 1570 can include a distal tip 1571 including one or more electrodes 1573 configured to electrically map and/or pace the septal wall SW. Accordingly, the first catheter 1570 can be used to determine a target site for implantation of the receiver-stimulator 1510 along the septal wall SW. In some embodiments, the first catheter 1570 can have a size of about 7 French or about 8 French. The first catheter 1570 and the first sheath 1572 can access the left ventricle LV via a transseptal or transaortic intravascular path.

Figure 15B illustrates the delivery system 1550 after advancing a puncture element 1576 (e.g., a needle) through the first catheter 1570 and into and through the septal wall SW (e.g., into the right ventricle RV).

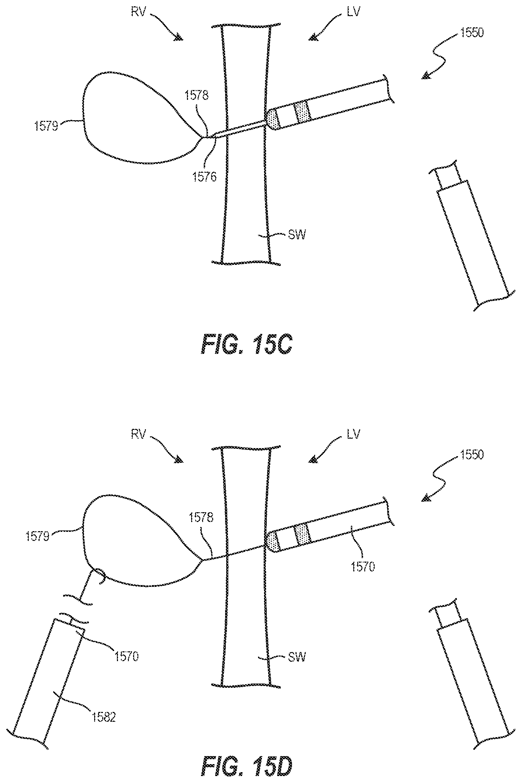

Figure 15C illustrates the delivery system 1550 after advancing a suture 1578 through the puncture element 1576 and into the right ventricle RV. In the illustrated embodiment, the suture 1578 forms a loop 1579 that is positioned in the right ventricle RV.

Figure 15D illustrates the delivery system 1550 after (i) withdrawing the puncture element 1576 (Figure 15C) through the first catheter 1570 and (ii) advancing a hook element 1580 through a second sheath 1582 and into the right ventricle RV. The second sheath 1582 can be positioned at least partially within the right ventricle RV or can be positioned proximally within the vasculature of the patient. As shown, the hook element 1580 can be used to capture the loop 1579 of the suture 1578 within the right ventricle RV.

Figure 15E illustrates the delivery system 1550 after (i) withdrawing the loop 1579 (Figure 15D) of the suture 1578 into the second sheath 1582 by withdrawing the hook element 1580 (Figure 15D) and (ii) withdrawing the first catheter 1570 through the first sheath 1572. Accordingly, at this stage the suture 1578 can span between the first and second sheaths 1572, 1582 while extending through the septal wall SW.

Figure 15F illustrates the delivery system 1550 during advancement of the receiver-stimulator 1510 over the suture 1578 and through the first sheath 1572. In some embodiments, the receiver-stimulator 1510 (e.g., the needle 1540) is attached to a distal end 1583 of the suture 1578. Accordingly, the receiver-stimulator 1510 can be advanced via the withdrawal of the suture 1578 through the second sheath 1582. In some embodiments, the first sheath 1572 can be advanced into the left ventricle LV before and/or during advancement of the receiver-stimulator 1510 through the first sheath 1572.

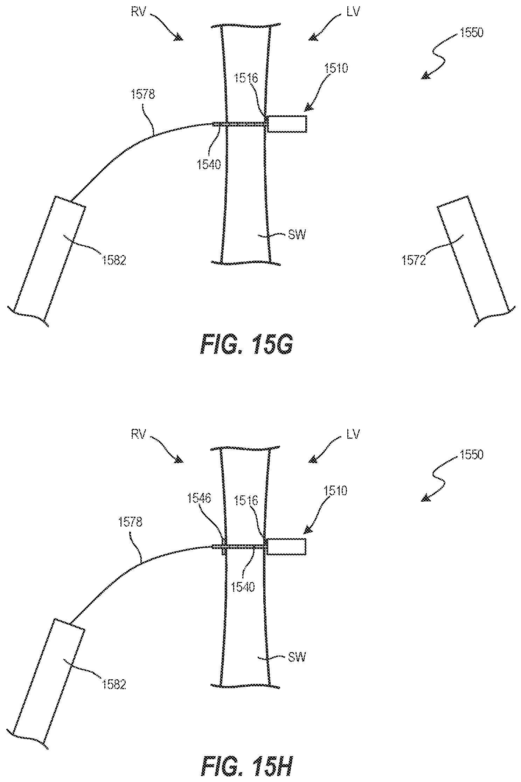

Figure 15G illustrates the delivery system 1550 after continued advancement of the receiver stimulator 1510 toward and into the septal wall SW. In some embodiments, continued withdrawal of the suture 1578 into the second sheath 1582 can pull the needle 1540 of the receiver-stimulator 1510 into and through the septal wall SW such that (i) the needle 1540 extends from the left ventricle LV into the right ventricle RV and (ii) the electrode 1516 of the receiver-stimulator 1510 is positioned against the septal wall SW within the left ventricle LV.

Figure 15H illustrates the delivery system 1550 after (i) advancement of the anchor mechanism 546 over the suture 1578 through the second sheath 1582, and onto the needle 1540 in the right ventricle RV (ii) withdrawal of the first sheath 1572 (Figure 15G). In some embodiments, a delivery catheter (not shown) can be used to advance the anchor member 1546 over the suture 1578 onto the needle 1540. In some embodiments, the anchor member 1546 can be attached to (e.g., screwed onto) the needle 1540 to firmly secure the electrode 1516 in contact with the septal wall SW via a compressive force on the septal wall SW.

Figures 15I illustrates an optional alignment stage that can be performed before installation of the anchor member 1546 (as shown in Figure 15H) in which a second catheter 1584 can be (i) advanced over the suture 1578 (obscured in Figure 15I) through the second sheath 1582 to engage the needle 1540 and (ii) then rotated to rotate the electrode 1516 to change the location of the electrode 1516 along the septal SW due to the eccentric or offset positioning of the electrode 1516 along the body 1512 of the receiver-stimulator 1510. In some embodiments, the second catheter 1584 can rotate the receiver-stimulator 1510 until the electrode 1516 is optimally positioned along the septal wall SW. Figure 15J, for example, illustrates the eccentrically-positioned electrode 1516 after it has been rotated to align with a target conductive structure CS, such as a bundle branch, within the septal wall SW.

Finally, Figure 15K illustrates the receiver-stimulator 1510 after removal of the delivery system 1550 (Figures 15A-15I) from the patient. At this stage, the receiver-stimulator 1510 remains at a target implant location along the septal wall SW. Referring to Figures 15H and 15K together, the suture 1578 can be released (e.g., cut) from the needle 1540 of the receiver-stimulator 1510, and the suture 1578 and the second sheath 1582 can be withdrawn from the patient.

### IV. Conclusion

The above detailed description of embodiments of the technology are not intended to be exhaustive or to limit the technology to the precise form disclosed above. Although specific embodiments of, and examples for, the technology are described above for illustrative purposes, various equivalent modifications are possible within the scope of the technology as those skilled in the relevant art will recognize. For example, although steps are presented in a given order, alternative embodiments can perform steps in a different order. The various embodiments described herein can also be combined to provide further embodiments.

From the foregoing, it will be appreciated that specific embodiments of the technology have been described herein for purposes of illustration, but well-known structures and functions have not been shown or described in detail to avoid unnecessarily obscuring the description of the embodiments of the technology. Where the context permits, singular or plural terms can also include the plural or singular term, respectively.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications can be made without deviating from the technology. Further, while advantages associated with some embodiments of the technology have been described in the context of those embodiments, other embodiments can also exhibit such advantages, and not all embodiments need necessarily exhibit such advantages to fall within the scope of the technology. Accordingly, the disclosure and associated technology can encompass other embodiments not expressly shown or described herein.

## Claims

1. A stimulation assembly (310) implantable within a heart of a patient, comprising:
a body (312);
circuitry positioned at least partially within the body (312) and configured to receive acoustic energy from an external source and to convert the acoustic energy to electrical energy;
an electrode (316) configured to receive the electrical energy; and
an anchor (314) coupled to the body (312) and configured to engage a septal wall of the heart such that (a) the body is positioned within a first ventricle of the heart that is separated from a second ventricle of the heart by the septal wall, (b) the anchor (314) pierces the septal wall and extends from the first ventricle to the second ventricle, and (c) the electrode (316) engages tissue of the septal wall, wherein the electrode (316) is further configured to deliver the electrical energy to the tissue of the septal wall.

2. The stimulation assembly of claim 1 wherein the anchor (314) has a corkscrew shape.

3. The stimulation assembly of claim 2 wherein the electrode (316) is positioned on the anchor.

4. The stimulation assembly of claim 3 wherein the electrode (316) is one of a pair of bipolar electrodes positioned on the anchor, and wherein the electrodes (316) are each configured to receive a portion of the electrical energy and to deliver the portion of the electrical energy to the tissue of the septal wall.

5. The stimulation assembly of claim 1 wherein the first ventricle is the left ventricle of the heart, wherein the body has a distal surface configured to be positioned adjacent the septal wall within the left ventricle, and wherein the anchor (314) comprises a needle (540) extending from the distal surface.

6. The stimulation assembly of claim 5 wherein the electrode (316) is one of a plurality of electrodes, wherein the electrodes (316) are positioned on the needle (540), and wherein each of the electrodes (316) is configured to receive a portion of the electrical energy and to deliver the portion of the electrical energy to the tissue of the septal wall.

7. The stimulation assembly of claim 5 or claim 6 wherein the electrodes (316) are linearly positioned along the needle (540), wherein the needle (540) is configured to be implanted within the tissue of the septal wall, and wherein the circuitry is further configured to selectively deliver the electrical energy to a target one of the electrodes (316).

8. The stimulation assembly of any one of claims 1-7 wherein the electrode (316) is one of a plurality of electrodes, wherein the electrodes (316) are positioned on the body, and wherein each of the electrodes (316) is configured to receive a portion of the electrical energy and to deliver the portion of the electrical energy to the tissue of the septal wall.

9. The stimulation assembly of claim 8 wherein the circuitry is further configured to selectively deliver the portions of the electrical energy to the electrodes (316) according to a selected stimulation pattern.

10. The stimulation assembly of any one of claims 1-9 wherein the first ventricle is the left ventricle of the heart, and wherein the second ventricle is the right ventricle of the heart.

11. The stimulation assembly of any one of claims 1-9 wherein the first ventricle is the right ventricle of the heart, and wherein the second ventricle is the left ventricle of the heart.

12. The stimulation assembly of claim 1 wherein:
the body (312) has a distal surface configured to be positioned adjacent the septal wall;
the anchor (314) is an elongate member extending from the distal surface and configured to extend through the septal wall from the first ventricle to a second ventricle of the heart; and
the stimulation assembly further comprising an anchor member configured to be secured to the elongate member within the second ventricle of the heart to secure the electrode (316) in contact with tissue of the septal wall.

13. The stimulation assembly of claim 12 wherein the anchor member and the distal surface of the body are configured to exert a compressive force against the septal wall, and wherein the electrode (316) is positioned at the distal surface of the body (312).

14. The stimulation assembly of claim 13 wherein the body has a longitudinal axis extending perpendicular to the distal surface and coincident with the elongate member, and wherein the electrode (316) is positioned away from the longitudinal axis.

15. The stimulation assembly of any one of claims 12 to 14 wherein:
the electrode (316) is positioned on the elongate member; or
the elongate member comprises an electrode material, wherein the stimulation assembly further comprises an insulative coating (1011) on the electrode material, and wherein the insulative coating has an opening that defines the electrode (316).

16. The stimulation assembly of any one of the preceding claims wherein the anchor (314) is configured to secure the electrode in position relative to the septal wall during tissue stimulation.

## Patentansprüche

1. Stimulationsbaugruppe (310), die innerhalb eines Herzens eines Patienten implantiert werden kann, Folgendes umfassend:
einen Körper (312);
eine Schaltung, die wenigstens teilweise innerhalb des Körpers (312) positioniert und dazu konfiguriert ist, akustische Energie von einer externen Quelle zu empfangen und die akustische Energie in elektrische Energie umzuwandeln;
eine Elektrode (316), die dazu konfiguriert ist, die elektrische Energie aufzunehmen; und
einen Anker (314), der mit dem Körper (312) gekoppelt und dazu konfiguriert ist, in eine Septumwand des Herzens einzugreifen, sodass (a) der Körper innerhalb eines ersten Ventrikels des Herzens positioniert ist, der von einem zweiten Ventrikel des Herzens durch die Septumwand getrennt ist, (b) der Anker (314) die Septumwand durchstößt und sich von dem ersten Ventrikel zu dem zweiten Ventrikel erstreckt, und (c) die Elektrode (316) in Gewebe der Septumwand eingreift, wobei die Elektrode (316) ferner dazu konfiguriert ist, die elektrische Energie an das Gewebe der Septumwand abzugeben.

2. Stimulationsbaugruppe nach Anspruch 1, wobei der Anker (314) eine Korkenzieherform aufweist.

3. Stimulationsbaugruppe nach Anspruch 2, wobei die Elektrode (316) auf dem Anker positioniert ist.

4. Stimulationsbaugruppe nach Anspruch 3, wobei die Elektrode (316) eine von einem Paar bipolarer Elektroden ist, die auf dem Anker positioniert sind, und wobei die Elektroden (316) jeweils dazu konfiguriert sind, einen Teil der elektrischen Energie aufzunehmen und den Teil der elektrischen Energie an das Gewebe der Septumwand abzugeben.

5. Stimulationsbaugruppe nach Anspruch 1, wobei der erste Ventrikel der linke Ventrikel des Herzens ist, wobei der Körper eine distale Oberfläche aufweist, die dazu konfiguriert ist, angrenzend an die Septumwand innerhalb des linken Ventrikels positioniert zu werden, und wobei der Anker (314) eine Nadel (540) umfasst, die sich von der distalen Oberfläche aus erstreckt.

6. Stimulationsbaugruppe nach Anspruch 5, wobei die Elektrode (316) eine von einer Mehrzahl von Elektroden ist, wobei die Elektroden (316) auf der Nadel (540) positioniert sind, und wobei jede der Elektroden (316) dazu konfiguriert ist, einen Teil der elektrischen Energie aufzunehmen und den Teil der elektrischen Energie an das Gewebe der Septumwand abzugeben.

7. Stimulationsbaugruppe nach Anspruch 5 oder 6, wobei die Elektroden (316) linear entlang der Nadel (540) positioniert sind, wobei die Nadel (540) dazu konfiguriert ist, innerhalb des Gewebes der Septumwand implantiert zu werden, und wobei die Schaltung ferner dazu konfiguriert ist, die elektrische Energie selektiv an eine Zielelektrode der Elektroden (316) abzugeben.

8. Stimulationsbaugruppe nach einem der Ansprüche 1-7, wobei die Elektrode (316) eine von einer Mehrzahl von Elektroden ist, wobei die Elektroden (316) auf dem Körper positioniert sind, und wobei jede der Elektroden (316) dazu konfiguriert ist, einen Teil der elektrischen Energie aufzunehmen und den Teil der elektrischen Energie an das Gewebe der Septumwand abzugeben.

9. Stimulationsbaugruppe nach Anspruch 8, wobei die Schaltung ferner dazu konfiguriert ist, die Teile der elektrischen Energie gemäß einem ausgewählten Stimulationsmuster selektiv an die Elektroden (316) abzugeben.

10. Stimulationsbaugruppe nach einem der Ansprüche 1-9, wobei der erste Ventrikel der linke Ventrikel des Herzens ist und wobei der zweite Ventrikel der rechte Ventrikel des Herzens ist.

11. Stimulationsbaugruppe nach einem der Ansprüche 1-9, wobei der erste Ventrikel der rechte Ventrikel des Herzens ist und wobei der zweite Ventrikel der linke Ventrikel des Herzens ist.

12. Stimulationsbaugruppe nach Anspruch 1, wobei:
der Körper (312) eine distale Oberfläche aufweist, die dazu konfiguriert ist, angrenzend an die Septumwand positioniert zu werden;
der Anker (314) ein längliches Element ist, das sich von der distalen Oberfläche erstreckt und dazu konfiguriert ist, sich durch die Septumwand von dem ersten Ventrikel zu einem zweiten Ventrikel des Herzens zu erstrecken; und
die Stimulationsbaugruppe ferner ein Ankerelement umfasst, das dazu konfiguriert ist, an dem länglichen Element innerhalb des zweiten Ventrikels des Herzens gesichert zu werden, um die Elektrode (316) in Kontakt mit dem Gewebe der Septumwand zu sichern.

13. Stimulationsbaugruppe nach Anspruch 12, wobei das Ankerelement und die distale Oberfläche des Körpers dazu konfiguriert sind, eine Druckkraft gegen die Septumwand auszuüben, und wobei die Elektrode (316) an der distalen Oberfläche des Körpers (312) positioniert ist.

14. Stimulationsbaugruppe nach Anspruch 13, wobei der Körper eine Längsachse aufweist, die sich senkrecht zu der distalen Oberfläche erstreckt und mit dem länglichen Element zusammenfällt, und wobei die Elektrode (316) von der Längsachse entfernt positioniert ist.

15. Stimulationsbaugruppe nach einem der Ansprüche 12 bis 14, wobei:
die Elektrode (316) auf dem länglichen Element positioniert ist; oder
das längliche Element ein Elektrodenmaterial umfasst, wobei die Stimulationsbaugruppe ferner eine isolierende Beschichtung (1011) auf dem Elektrodenmaterial umfasst, und wobei die isolierende Beschichtung eine Öffnung aufweist, die die Elektrode (316) definiert.

16. Stimulationsbaugruppe nach einem der vorhergehenden Ansprüche, wobei der Anker (314) dazu konfiguriert ist, die Elektrode während der Gewebestimulation in ihrer Position relativ zu der Septumwand zu sichern.

## Revendications

1. Ensemble de stimulation (310) implantable dans le cœur d'un patient, comprenant :
un corps (312) ;
des circuits positionnés au moins partiellement au sein du corps (312) et configurés pour recevoir de l'énergie acoustique d'une source externe et pour convertir l'énergie acoustique en énergie électrique ;
une électrode (316) configurée pour recevoir l'énergie électrique ; et
une ancre (314) couplée au corps (312) et configurée pour entrer en prise avec une paroi septale du cœur de sorte que (a) le corps est positionné au sein d'un premier ventricule du cœur qui est séparé d'un deuxième ventricule du cœur par la paroi septale, (b) l'ancre (314) perce la paroi septale et s'étend du premier ventricule au deuxième ventricule, et (c) l'électrode (316) entre en prise avec le tissu de la paroi septale, dans lequel l'électrode (316) est configurée en outre pour délivrer l'énergie électrique au tissu de la paroi septale.

2. Ensemble de stimulation selon la revendication 1 dans lequel l'ancre (314) a la forme d'un tire-bouchon.

3. Ensemble de stimulation selon la revendication 2 dans lequel l'électrode (316) est positionnée sur l'ancre.

4. Ensemble de stimulation selon la revendication 3 dans lequel l'électrode (316) est l'une d'une paire d'électrodes bipolaires positionnées sur l'ancre, et dans lequel les électrodes (316) sont chacune configurées pour recevoir une partie de l'énergie électrique et pour délivrer la partie de l'énergie électrique au tissu de la paroi septale.

5. Ensemble de stimulation selon la revendication 1 dans lequel le premier ventricule est le ventricule gauche du cœur, dans lequel le corps a une surface distale configurée pour être positionnée adjacente à la paroi septale au sein du ventricule gauche, et dans lequel l'ancre (314) comprend une aiguille (540) s'étendant à partir de la surface distale.

6. Ensemble de stimulation selon la revendication 5 dans lequel l'électrode (316) est l'une d'une pluralité d'électrodes, dans lequel les électrodes (316) sont positionnées sur l'aiguille (540), et dans lequel chacune des électrodes (316) est configurée pour recevoir une partie de l'énergie électrique et pour délivrer la partie de l'énergie électrique au tissu de la paroi septale.

7. Ensemble de stimulation selon la revendication 5 ou la revendication 6 dans lequel les électrodes (316) sont positionnées de manière linéaire le long de l'aiguille (540), dans lequel l'aiguille (540) est configurée pour être implantée au sein du tissu de la paroi septale, et dans lequel les circuits sont en outre configurés pour délivrer sélectivement l'énergie électrique à une électrode cible parmi les électrodes (316).

8. Ensemble de stimulation selon l'une quelconque des revendications 1 à 7 dans lequel l'électrode (316) est l'une d'une pluralité d'électrodes, dans lequel les électrodes sont positionnées sur le corps, et dans lequel chacune des électrodes (316) est configurée pour recevoir une partie de l'énergie électrique et pour délivrer la partie de l'énergie électrique au tissu de la paroi septale.

9. Ensemble de stimulation selon la revendication 8 dans lequel les circuits sont configurés en outre pour délivrer sélectivement les parties de l'énergie électrique aux électrodes (316) selon un motif de stimulation sélectionné.

10. Ensemble de stimulation selon l'une quelconque des revendications 1 à 9 dans lequel le premier ventricule est le ventricule gauche du cœur, et dans lequel le deuxième ventricule est le ventricule droit du cœur.

11. Ensemble de stimulation selon l'une quelconque des revendications 1 à 9 dans lequel le premier ventricule est le ventricule droit du cœur, et dans lequel le deuxième ventricule est le ventricule gauche du cœur.

12. Ensemble de stimulation selon la revendication 1 dans lequel :
le corps (312) a une surface distale configurée pour être positionnée adjacente à la paroi septale ;
l'ancre (314) est un élément allongé s'étendant à partir de la surface distale et configurée pour s'étendre à travers la paroi septale à partir du premier ventricule jusqu'à un deuxième ventricule du cœur ; et
l'ensemble de stimulation comprenant en outre un élément d'ancre configuré pour être fixé à l'élément allongé au sein du deuxième ventricule du cœur pour fixer l'électrode (316) en contact avec du tissu de la paroi septale.

13. Ensemble de stimulation selon la revendication 12 dans lequel l'élément d'ancre et la surface distale du corps sont configurés pour exercer une force de compression contre la paroi septale, et dans lequel l'électrode (316) est positionnée au niveau de la surface distale du corps (312).

14. Ensemble de stimulation selon la revendication 13 dans lequel le corps a un axe longitudinal s'étendant perpendiculairement à la surface distale et coïncident avec l'élément allongé, et dans lequel l'électrode (316) est positionnée à l'écart de l'axe longitudinal.

15. Ensemble de stimulation selon l'une quelconque des revendications 12 à 14 dans lequel :
l'électrode (316) est positionnée sur l'élément allongé ; ou
l'élément allongé comprend un matériau d'électrode, dans lequel l'ensemble de stimulation comprend en outre un revêtement isolant (1011) sur le matériau d'électrode, et dans lequel le revêtement isolant a une ouverture qui définit l'électrode (316).

16. Ensemble de stimulation selon l'une quelconque des revendications précédentes dans lequel l'ancre (314) est configurée pour fixer l'électrode en position par rapport à la paroi septale pendant la stimulation de tissu.
